# EUROPEAN PATENT SPECIFICATION

(11) **EP 1 003 708 B1**
(45) Date of publication and mention of the grant of the patent: **12.06.2002**
(21) Application number: 98943187.9
(22) Date of filing: 12.08.1998
(51) Int. Cl.: C07C 51/43, C07C 63/46

(54) **IMPROVED PROCESS FOR SEPARATING PURE TEREPHTHALIC ACID**
VERFAHREN ZUM SCHEIDEN VON REINER TEREPHTHALSÄURE
PROCEDE AMELIORE DE SEPARATION D'ACIDE TEREPHTALIQUE PUR

(30) Priority: 15.08.1997 GB 9717251
(43) Date of publication of application: 31.05.2000
(73) Proprietor: E.I. DU PONT DE NEMOURS AND COMPANY, Wilmington Delaware 19898 (US)
(72) Inventor: BICKHAM, David, Robert, Cleveland TS14 7LX (GB)
(74) Representative: Jones, Alan John
(86) International application number: US9816691
(87) International publication number: WO9908990

(56) References cited:
- WO-A-93/24440

## Description

This application claims benefit of GB Provisional Application No. 9717251.4, filed August 15, 1997.

### BACKGROUND OF THE INVENTION

The present invention relates to an improved process for separating pure terephthalic acid crystals from a slurry which contains such crystals along with dissolved impurities using a centrifuge, and, more particularly, to a process improvement whereby a carrier fluid is introduced into the centrifuge continuously and simultaneously with the slurry with the result that a higher proportion of impurities are removed from the pure terephthalic acid crystals than could have been achieved otherwise.

The production of pure terephthalic acid (PTA) involves oxidising para-xylene to form crude terephthalic acid (TA) which contains a number of impurities, particularly 4-carboxybenzaldehyde (4-CBA), and then purifying the crude TA. Crude TA crystals are dissolved in water at elevated pressure and temperature, and the solution is subjected to hydrogenation in the presence of a Group VIII noble metal hydrogenation catalyst. The purified acid is recovered by crystallizing the acid from the hydrogen treated aqueous solution, i.e., the pure plant mother liquor (PPML). The principal impurities, which are p-toluic acid derived from the compound 4-carboxybenzaldehyde and unidentified color bodies, along with some other organic components, such as benzoic acid and residual terephthalic acid, remain dissolved in the aqueous solution. The pure TA crystals are then separated from the pure plant mother liquor in one or more centrifuges, usually at elevated pressure. The present invention results in the isolation of pure TA crystals having lower levels of impurities, particularly p-toluic acid, than could have been isolated otherwise using high pressure centrifuges.

### SUMMARY OF THE INVENTION

The present invention is an improved process for separating pure terephthalic acid crystals from a slurry which contains such crystals along with dissolved impurities using a centrifuge. The improvement comprises introducing steam, optionally in the presence on an inert gas, such as nitrogen, as a carrier fluid into the centrifuge continuously and simultaneously with the slurry with the result that a higher proportion of the impurities, particularly p-toluic acid, are removed from the pure terephthalic acid crystals than could have been achieved otherwise. The centrifuges contemplated for use according to the invention typically include means for introducing the slurry into the centrifuge, along with a liquid and a solids output means. A difference in pressure is typically maintained between the liquid output means and the centrifuge and the solids output means of about 1-10 kPa. The separation of solid pure acid crystals from pure plant mother liquor is achieved according to the invention by introducing steam, optionally in the presence of an inert gas, such as, for example, nitrogen, into the centrifuge simultaneously and continuously with the slurry. The carrier fluid moves through the centrifuge and exits therefrom with separated pure plant mother liquor via the liquid output means. The recovered PTA crystals have an unexpectedly low concentration of p-toluic acid than PTA crystals produced without the use of a carrier fluid.

### BRIEF DESCRIPTION OF THE DRAWINGS

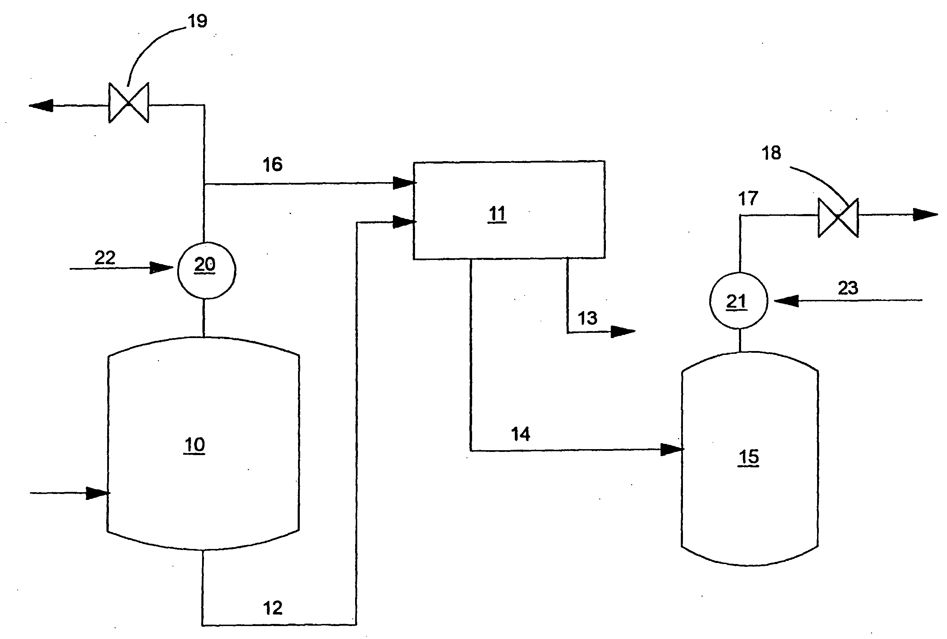

Fig. 1 is a simplified schematic diagram of a separation process according to the invention.

### DETAILED DESCRIPTION

In the purification stage of typical commercial processes for producing pure terephthalic acid, i.e., PTA,, crude terephthalic acid crystals are dissolved in water at elevated pressure and temperature, and the solution is subjected to hydrogenation in the presence of a Group VIII noble metal hydrogenation catalyst. The purified acid is recovered by crystallizing the acid from the hydrogen treated aqueous solution and then separating the pure crystals from the solution using one or more high pressure centrifuges. The separation process can be conducted batch-wise, although the process is usually carried out on a continuous basis. The principal impurities which are present in the hydrogen treated aqueous solution are p-toluic acid derived from the compound 4-carboxybenzaldehyde and various color bodies, along with some other organic components, such as benzoic acid and residual terephthalic acid. These impurities for the most part remain dissolved in the aqueous solution during the separation process. The aqueous solution which remains after separation of the pure TA crystals is referred to as "pure plant mother liquor", i.e., PPML.

The invention resides in the discovery that pure TA crystals can be separated from a pure plant mother liquor with a substantially lower concentration of p-toluic acid than could be achieved otherwise by introducing steam, optionally in the presence on an inert gas, such as nitrogen, as a carrier fluid into the centrifuge continuously and simultaneously with the slurry. Any carrier fluid which is inert with respect to the pure TA and PPML is contemplated for use in carrying out the invention.

In practice, the slurry of pure TA crystals passes into the centrifuge from a crystallizer, in which the temperature and pressure of the incoming slurry has been reduced thereby producing steam. This, in turn, can be a source of steam for practicing the invention. Residual solids which may be present in the steam as it is vented from the crystallizer can be removed before it is introduced into the centrifuge. A water spray in the crystalliser steam vent line is suitable for this purpose.

The flow of steam, i.e., carrier fluid, through the centrifuge can be adjusted by valves.

In practice, the pressure in the liquid output means from the centrifuge is less than the pressure in the centrifuge, and this pressure differential provides a convenient method for inducing a flow of the carrier fluid through the centrifuge and facilitates its removal via the liquid output means such that said carrier fluid flows into and through the centrifuge continuously and simultaneously with the pure TA slurry. The flow of carrier fluid through the centrifuge and into the liquid output means is responsive to the reduction of pressure in the liquid output means relative to the pressure of the centrifuge.

In practice, a water spray can be directed into the liquid output means, e.g. into the vessel for containing the liquid or into the vapor line which exits such a vessel which, in turn, acts to reduce foaming in the liquid output means and thereby reduce carry over of any solids into the vapor lines.

An embodiment of the process of the invention will now be described with reference to Fig. 1, which is a simplified schematic diagram of the relevant portion of a process for purifying crude terephthalic acid.

The purification of crude terephthalic acid to produce PTA is carried out largely to remove 4-carboxy benzaldehyde (4-CBA). 4-CBA is an undesirable impurity in a PTA product because it can detrimentally affect the molecular weight of the polyester, i.e., poly(ethylene terephthalate) made therefrom. A typical specification for commercially produced PTA specifies a 4-CBA concentration of less than 20 ppm. In a typical process for removing 4-CBA, the 4-CBA is catalytically hydrogenated to p-toluic acid, which is then removed by dissolution in water, from which solution PTA is crystallized out.

The crystallization process is multi-stage and operates at elevated temperatures and pressure. The final crystallizer is typically operated at a temperature in the range of from 140° to 155°C and at a pressure in the range of from 400 to 500 kPa, although higher and lower temperatures and pressures can be used with satisfactory results.

Referring now to the drawing, final crystallizer 10 produces a slurry of PTA crystals in the pure plant mother liquor. The slurry is pumped via line 12 to centrifuge 11. In practice PTA crystals are separated as a solid and recovered from the slurry in one or more centrifuges 11. Centrifuge 11 has a discharge chute for continuously removing solids via line 13 and a liquid runoff line 14 which runs to mother liquor storage tank 15, collectively "liquid output means". Spray 21 sprays water into the mother liquor storage tank 15 which reduces foaming and, in turn, improves the flow of mother liquor from the centrifuge. Solid PTA, recovered as a "cake" from the centrifuge, is re-slurried with water and re-centrifuged and then dried to form a final PTA crystalline product. The pressure in mother liquor storage tank 15 and line 14 is low relative to the pressure in centrifuge 11. Steam flows continuously from the crystallizer exit via transfer line 16 into centrifuge 11 and exits through line 14 with pure plant mother liquor. As shown, steam is generated in crystallizer 10 during operation by reducing the temperature and pressure of the incoming aqueous slurry of pure terephthalic acid. Hot water spray 20 is present as shown to remove residual solids to the extent they may be present in the vapor line being carried over from the crystallizer. The steam is returned to a steam venting system via line 17. The flow of steam from crystallizer 10 can be controlled as shown by valves 18 & 19. Valve 19 controls the flow of steam to vent header.

The advantage of using a carrier fluid such as steam according to the invention is compared to a conventional process and illustrated in the following examples.

### Example

A terephthalic acid purification process was operated using a crude terephthalic acid feed obtained from the oxidation of p-xylene. Crude terephthalic acid (CTA) was purified by dissolving it in water to produce an aqueous solution which was then hydrogenated in the presence of a hydrogenation catalyst whereby 4-CBA was reduced to p-toluic acid. The hydrogenated CTA was then passed through a series of crystallizers in which pure terephthalic acid crystallized out of solution leaving the p-toluic acid and other impurities in solution. The 4-CBA contained in the crude TA feed was measured at different points in time and the p-toluic acid content of the purified TA was also measured. Since the process involved conversion of 4-CBA to p-toluic acid, as the 4-CBA content of the feed increased, the amount of dissolved p-toluic acid which must be removed in the centrifuge also increased.

Table 1 shows typical levels of 4-CBA in the crude CTA feed and the p-toluic acid levels in the resulting PTA product using a centrifuge with and without introduction of a steam carrier fluid. The mother liquor output tank, i.e., "liquid output means" was maintained at a reduced pressure relative to the centrifuge. All of the PTA product contained less than 150 ppm of p-toluic acid. Before introducing steam as carrier fluid, low levels of p-toluic acid were achieved by reducing the amount of 4-CBA produced in the oxidation stage of the process. However, in doing so, some of the p-xylene feedstock and acetic acid solvent was consumed in undesirable side reactions, which, in turn, can substantially increase the cost of operating the process on a commercial scale.

**Table 1**

| | Before steam | | | After steam | | |
|---|---|---|---|---|---|---|
| 4-CBA in feed (%w/w) | 0.20 | 0.17 | 0.18 | 0.22 | 0.21 | 0.23 |
| p-toluic acid in PTA product (ppm) | 145 | 130 | 140 | 125 | 107 | 117 |

The p-toluic acid levels in the PTA product after introducing steam into the centrifuge as a carrier fluid according to the invention are significantly lower, although the levels of 4-CBA in the feed are generally higher than runs measured without the carrier fluid. Thus, separation of p-toluic acid has been improved using a carrier fluid, e.g., steam, according to the invention.

## Claims

1. In a process for purifying crude terephthalic acid comprising the steps of:
a) dissolving said crude terephthalic acid in water at elevated temperature and pressure,
b) passing the resulting solution with hydrogen over a hydrogenation catalyst whereby 4-CBA is converted to p-toluic acid,
c) reducing the temperature of the hydrogenated solution whereby pure terephthalic acid crystals crystallize out of solution to form a slurry, and
d) separating the pure terephthalic acid crystals from the slurry using centrifugal force in a centrifuge, the improvement comprising
e) introducing a carrier fluid into the centrifuge continuously and simultaneously with the slurry.

2. The process of Claim 1 in which the carrier fluid is steam.

3. An improved process for separating pure terephthalic acid crystals from a slurry which contains such crystals along with dissolved impurities using centrifugal force in a centrifuge wherein the improvement comprises introducing steam as a carrier fluid into the centrifuge continuously and simultaneously with the slurry.

4. The process of claim 2 in which said steam is accompanied by an additional inert gas.

5. The process of Claim 3 in which the inert gas is nitrogen.

## Patentansprüche

1. Verfahren zum Reinigen roher Terephthalsäure, umfassend die Stufen von:
a) Lösen der rohen Terephthalsäure in Wasser bei erhöhter Temperatur und Druck,
b) Leiten der sich ergebenden Lösung mit Wasserstoff über einen Hydrierungskatalysator, wobei 4-CBA zu p-Toluylsäure umgewandelt wird,
c) Reduzieren der Temperatur der hydrierten Lösung, wobei reine Terephthalsäurekristalle aus Lösung kristallisieren unter Bilden einer Aufschlämmung und
d) Trennen der reinen Terephthalsäurekristalle von der Aufschlämmung unter Verwenden von Zentrifugationskraft in einer Zentrifuge, **gekennzeichnet durch**
e) Einführen eines Trägerfluidums in die Zentrifuge kontinuierlich und gleichzeitig mit der Aufschlämmung.

2. Verfahren nach Anspruch 1, bei dem das Trägerfluidum Dampf ist.

3. Verbessertes Verfahren zum Trennen reiner Terephthalsäurekristalle von einer Aufschlämmung, die derartige Kristalle neben gelösten Verunreinigungen enthält, unter Verwenden von Zentrifugationskraft in einer Zentrifuge, **gekennzeichnet durch** Einführen von Dampf als ein Trägerfluidum in die Zentrifuge kontinuierlich und gleichzeitig mit der Aufschlämmung.

4. Verfahren nach Anspruch 2, bei dem der Dampf von einem zusätzlichen Inertgas begleitet wird.

5. Verfahren nach Anspruch 3, bei dem das Inertgas Stickstoff ist.

## Revendications

1. Procédé pour la purification d'acide téréphtalique brut comprenant les étapes consistant:
a) à dissoudre ledit acide téréphtalique brut dans de l'eau à une température et à une pression élevées,
b) à faire passer la solution résultante avec de l'hydrogène au-dessus d'un catalyseur d'hydrogénation, en conséquence de quoi le 4-CBA est converti en acide p-toluique,
c) à réduire la température de la solution hydrogénée, en conséquence de quoi des cristaux d'acide téréphtalique pur se séparent par cristallisation de la solution pour former une suspension épaisse, et
d) à séparer les cristaux d'acide téréphtalique pur de la suspension épaisse en utilisant la force centrifuge dans une centrifugeuse, l'amélioration comprenant:
e) l'introduction d'un fluide porteur dans la centrifugeuse en continu et simultanément à la suspension épaisse.

2. Procédé suivant la revendication 1, dans lequel le fluide porteur est de la vapeur.

3. Procédé amélioré pour la séparation de cristaux d'acide téréphtalique pur d'une suspension épaisse qui contient de tels cristaux accompagnés d'impuretés dissoutes en utilisant la force centrifuge dans une centrifugeuse, dans lequel l'amélioration comprend l'introduction de vapeur en tant que fluide porteur dans la centrifugeuse en continu et simultanément à la suspension épaisse.

4. Procédé suivant la revendication 2, dans lequel ladite vapeur est accompagnée d'un gaz inerte supplémentaire.

5. Procédé suivant la revendication 3, dans lequel le gaz inerte est de l'azote.
